# EUROPÄISCHE PATENTSCHRIFT

(11) **EP 3 238 757 B1**
(45) Veröffentlichungstag und Bekanntmachung des Hinweises auf die Patenterteilung: **14.06.2023**
(21) Anmeldenummer: 17167785.9
(22) Anmeldetag: 24.04.2017
(51) Int. Cl.: A61M 1/16

(54) **GERÄT ZUR EXTRAKORPORALEN BLUTBEHANDLUNG MIT KONZENTRATWECHSEL**
DEVICE FOR EXTRACORPOREAL BLOOD TREATMENT WITH CONCENTRATE CHANGE
APPAREIL POUR LE TRAITEMENT EXTRACORPOREL DU SANG AU MOYEN DE CHANGEMENT DE CONCENTRÉ

(30) Priorität: 25.04.2016 DE 102016107589
(43) Veröffentlichungstag der Anmeldung: 01.11.2017
(73) Patentinhaber: B. Braun Avitum AG, 34212 Melsungen (DE)
(72) Erfinder: Riemenschneider, Heiko, 34327 Wagenfurth (DE)
(74) Vertreter: Winter, Brandl - Partnerschaft mbB

(56) Entgegenhaltungen:
- EP-A1- 0 714 668
- EP-A2- 0 160 272
- DE-A1-102013 102 914
- DE-C1- 19 824 057
- US-A1- 2009 012 455
- US-A1- 2016 051 949

## Beschreibung

Die Erfindung betrifft ein Gerät zur extrakorporalen Blutbehandlung mit Konzentratwechsel und bezieht sich insbesondere auf ein Gerät zur extrakorporalen Blutbehandlung, bei dem während einer laufenden Behandlung eigensteuerbar und/oder automatisch ein Wechsel von einem ersten Konzentrat zu einem zweiten Konzentrat erfolgen kann.

Ein bekanntes Gerät für extrakorporale Blutbehandlung, wie beispielsweise ein Dialysegerät bzw. eine Dialysemaschine, umfasst zumindest eine Behandlungseinheit, wie beispielsweise einen Dialysator bzw. eine Dialysatorvorrichtung, oder einen Filter, Ultrafilter oder Plasmafilter einer andersartigen Filtereinheit mit einer semipermeablen Membran, welche die Behandlungseinheit in zwei Kammern trennt. Ein extrakorporaler Blutkreislauf ermöglicht einen Fluss von aus einem Patienten entnommenem Blut durch die erste Kammer hindurch zurück zum Patienten. Gleichzeitig fließt gegenläufig dazu eine Dialysierflüssigkeit (Behandlungsflüssigkeit) durch einen geeignet ausgelegten Kreislauf über die zweite Kammer. Das bekannte Gerät beinhaltet darüber hinaus eine Infusionsleitung für ein Substitutionsfluid, eine einlassseitig mit der zweiten Kammer verbundene Fluideinlassleitung und eine auslassseitig mit der zweiten Kammer verbundene Fluidauslassleitung.

In solchen Geräten zur extrakorporalen Blutbehandlung werden (flüssige oder trockene) Konzentrate in Kanistern oder Containern als Grundsubstanz für die Herstellung der Dialysierflüssigkeit eingesetzt. Dazu zählen beispielsweise Acetat-Hämodialysekonzentrate, saure Hämodialysekonzentrate, Bicarbonat-Konzentrate, Natriumhyrogencarbonatpulver und dergleichen, um nur einige zu nennen. Sie enthalten Elektrolyte wie Natrium, Chlorid, Kalium, Kalzium und Magnesium. Von dem Dialysegerät werden die Konzentrate mit gereinigtem Wasser auf physiologische Werte verdünnt, bevor sie als Dialysierflüssigkeit eingesetzt werden können und den Dialysator durchströmen.

Die richtige Auswahl von Konzentraten zur Anpassung der Dialysierflüssigkeit, um die Individualisierung der Therapieparameter optimal und den Patienten unterstützend anzupassen, ist bislang eine Herausforderung für den Anwender.

Speziell mit steigender Anzahl an Hämodiafiltrations-Therapien wird ein manuelles Wechseln der Konzentrate von Hand und mit kurzer Therapieunterbrechung immer häufiger angewendet. Dies bindet Personal und kann nicht immer exakt zum vorgeschriebenen Zeitpunkt der laufenden Therapie durchgeführt werden. Infolge solcher technischer und personeller Einschränkungen wird häufig ein Konzentrat gewählt, welches nicht den Therapieverlauf nicht optimal unterstützt.

Bekannte Maßnahmen zum Anpassen des Behandlungsverlaufs bestehen darin, während einer laufenden Therapie oder Blutbehandlung nach Vorgabe eines Arztes das Konzentrat zu einem vorbesprochenen Zeitpunkt zu wechseln oder ein Konzentrat zu wählen, das über den Verlauf der Therapie verwendet werden kann, ohne das es zu einer kritischen Absenkung z.B. des Kaliumpegels kommt oder, falls es in der laufenden Therapie zu Komplikationen durch einen zu niedrigen Kaliumpegel kommt, das Konzentrat in Folge manuell zu wechseln. Ferner sind Systeme bekannt, die das Konzentrat aus drei Komponenten aufbereiten (so genannter "three mix"), die jedoch technisch sehr aufwendig, teuer und in der Überwachung risikobehaftet sind. Des Weiteren werden in einer derartigen Anwendung auch andere Elektrolyte limitiert, da diese gleichzeitig mit angehoben und/oder gesenkt werden.

Zudem offenbart die Druckschrift US2016/0051949A1 ein Verfahren zur Online-Herstellung einer Behandlungsflüssigkeit bei extrakorporaler Blutbehandlung in einer Dialysemaschine, bei der Wasser über eine Hauptleitung zuführbar und Konzentrate entlang der Hauptleitung nacheinander (dem Wasser) hinzufügbar sind. Aufgrund einer Rückkopplung bzw. Regelung kann dabei (zu einem bestimmten Zeitpunkt automatisch) von einer Steuerung einer ersten Pumpe für das erste Konzentrat auf eine Steuerung einer zweiten Pumpe für das zweite Konzentrat übergegangen werden.

Die Druckschrift DE 19824057 C1 betrifft ebenfalls die Herstellung einer Behandlungsflüssigkeit bei extrakorporaler Blutbehandlung bzw. für eine Dialysemaschine, bei der zur individuellen Einstellung der Zusammensetzung der Dialysierflüssigkeit auch während einer Dialysebehandlung eine kontinuierliche Individualeinstellung während der Dialysebehandlung durch laufende Herstellung von kleinen Dialysierflüssigkeitskonzentratmengen, die sich ihrerseits wieder in ihrer Zusammensetzung zueinander ändern können, erfolgt. Hierbei beinhalten die in Zwischenspeichern zu mischenden Komponenten kein Bicarbonat-Konzentrat.

Aus der Druckschrift EP 0 160 272 A2 ist zudem eine Hämodialysevorrichtung bekannt, bei der die Dialysierflüssigkeit aus wenigstens einem Konzentrat mit Hilfe einer ersten und zweiten Pumpe hergestellt wird, wobei die erste Pumpe so viel Konzentrat fördert, dass eine Basisdialysierflüssigkeit eines bestimmten unteren Werts erzeugt wird. Die zweite Konzentratpumpe fördert zusätzliches Konzentrat in einem begrenzten Bereich.

Die US 2009/0012455 A1 offenbart eine Dialysevorrichtung für insbesondere eine automatische Peritoneale Dialyse (ADP) und für eine Hämodiafiltration (HDF). Bei dieser Vorrichtung wird mittels einer induktiven Koppelsignatur und einer bekannten spezifischen Leitfähigkeit eines Konzentrats dieses Konzentrat entsprechend überprüft.

Die vorstehend beschriebenen bekannten Techniken zwingen bislang den Anwender in eine nicht optimal patientenbezogene Anwendung. Der personelle Aufwand, das Risiko und die in der Maschine durch einen Konzentratwechsel bedingte zeitliche Therapieunterbrechung ermöglichen lediglich eine akzeptable Anwendung anstelle einer optimalen und patientenbezogenen Therapie.

Der Erfindung liegt daher als eine Aufgabe zugrunde, die vorgenannten Probleme zu überwinden und ein Gerät zur extrakorporalen Blutbehandlung bereitzustellen, welches dazu eingerichtet ist, das Therapieergebnis zu verbessern und gleichzeitig den Anwender zu entlasten.

Außerdem soll die Erfindung eine sichere Anwendung gewährleisten und gleichzeitig ermöglichen, einen Patienten belastungsfreier und mit besserem Behandlungsergebnis zu behandeln.

Diese Aufgabe wird erfindungsgemäß durch ein Gerät zur extrakorporalen Blutbehandlung mit den Merkmalen des Anspruchs 1 gelöst. Vorteilhafte Weiterbildungen der Erfindung sind Gegenstand der beigefügten Unteransprüche.

Der Erfindung liegt als eine allgemeine Idee zugrunde, den Behandlungsverlauf des Patienten durch einen Konzentratwechsel während einer laufenden Blutbehandlung oder Therapie anzupassen, vorzugsweise automatisch anzupassen. Dafür ist bei einer Maschine zur extrakorporalen Blutbehandlung eine Konzentrat zuführende Vorrichtung dazu ausgebildet, nach einer bestimmten Zeitspanne von einem ersten Konzentrat auf zumindest ein zweites Konzentrat zu wechseln oder umzuschalten. Das Konzentrat kann zumindest in zwei Kanistern (Behältern, Beuteln, Kartuschen) vorgehalten werden. Der zuständige Arzt vermerkt in einem Therapieprotokoll die Art der Konzentrate und den oder die Wechselzeitpunkt(e). Falls während einer erstmaligen Therapie ein konkreter Wechsel- oder Umschaltzeitpunkt noch nicht bekannt ist bzw. feststeht, kann das Therapiesystem dazu eingerichtet sein, diesen automatisch von Therapie zu Therapie zu lernen. In anderen Worten kann ein Wechselzeitpunkt als Vorgabe für eine nachfolgende Therapie mitgeschrieben werden. Die Hardware kann dazu ausgelegt sein, bei Bedarf einen vorhandenen Bicarbonatanschluss als zweiten Säurezugang zu nutzen. Die automatische Umschaltung und die Prüfung der Konzentrate können softwaregestützt erfolgen.

Der Arzt wird dadurch in die Lage versetzt, auf die insbesondere am Anfang der Therapie stark verschobenen Elektrolytwerte gesondert einzugehen. Da sich die Werte im Blutbild schnell ändern können, kann der Arzt durch die Wahl des zweiten Konzentrates und des Zeitpunkts des Einsatzes mit einem angepassten Diffusionsdruck den Therapiefortschritt der Behandlung besser vorbereiten und entsprechend schnell anpassen.

Die Erfindung stellt somit eine technische Lösung bereit, die den Anwender automatisch und sicher unterstützt. Es muss keine Therapieunterbrechung, die durch den Wechsel von Konzentraten entsteht, in Kauf genommen werden. Auch eine zeitliche Verschiebung des Therapieendes entfällt.

Im Einzelnen wird die Aufgabe gelöst durch ein Gerät zur extrakorporalen Blutbehandlung, beinhaltend: zumindest einen ersten Säurekonzentratanschluss, der dazu angeordnet ist, zumindest ein erstes Säurekonzentrat (AC1) als Grundlage für eine Erzeugung einer Dialysierflüssigkeit in das Gerät zur extrakorporalen Blutbehandlung zu leiten; zumindest einen zweiten Säurekonzentratanschluss, der dazu angeordnet ist, zumindest ein zweites Säurekonzentrat (AC2) als Grundlage für eine Erzeugung einer Dialysierflüssigkeit in das Gerät zur extrakorporalen Blutbehandlung zu leiten; wobei das Gerät zur extrakorporalen Blutbehandlung dazu eingerichtet ist, während einer laufenden Blutbehandlung zu einem vorbestimmten Zeitpunkt oder nach einer vorbestimmten Zeitspanne von einer Zufuhr des zumindest ersten Säurekonzentrats (AC1) auf eine Zufuhr des zumindest zweiten Säurekonzentrats (AC2) umzuschalten, wobei ein Bicarbonatanschluss des Geräts derart als der erste oder der zweite Konzentratanschluss konfiguriert ist, dass dem Gerät über den Bicarbonatanschluss das erste oder das zweite Säurekonzentrat (AC1, AC2) zuführbar ist, und Bicarbonat (BI) separat von dem Bicarbonatanschluss zuführbar ist.

Dabei ist das Gerät dazu konfiguriert, einen Konzentratwechselzeitpunkt während einer laufenden Behandlung zu protokollieren und als Vorgabezeitpunkt für mindestens eine nachfolgende Behandlung zu speichern und bereitzustellen.

Bevorzugt ist das Gerät dazu angeordnet, die Umschaltung von der Zufuhr des zumindest ersten Konzentrats auf die Zufuhr des zumindest zweiten Konzentrats automatisch durchzuführen.

Bevorzugt sind das erste Konzentrat und/oder das zweite Konzentrat in Form eines Flüssigkonzentratvorrats oder Trockenkonzentratvorrats in jeweils einem ersten und einem zweiten Konzentratgebinde vorhaltbar, wobei das erste Konzentratgebinde konzentratdurchlässig mit dem ersten Konzentratanschluss und das zweite Konzentratgebinde konzentratdurchlässig mit dem zweiten Konzentratanschluss koppelbar sind.

Bevorzugt ist zusätzlich zu bestehenden Konzentratanschlüssen mindestens ein weiterer Konzentratanschluss als der erste oder der zweite Konzentratanschluss angeordnet.

Bevorzugt ist eine Vielzahl von Arten verwendbarer erster und zweiter Konzentrate vorab in einer Speichereinrichtung des Geräts gespeichert, und sind an dem Gerät zumindest das erste und zumindest das zweite Konzentrat aus der dem Gerät bekannten Vielzahl von Arten verwendbarer Konzentrate auswählbar.

Bevorzugt ist das Gerät dazu konfiguriert, zumindest eine patientenbezogen korrekte Auswahl von Konzentraten, Mischungsverhältnisse derselben und eine Anschlussbelegung durch ausgewählte Konzentrate über eine Mehrzahl von Behandlungen hinweg zu lernen und zu speichern.

Bevorzugt ist dann, wenn geräteseitig eine Änderung eines konzentratbezogenen Mischungsverhältnisses erfasst wird, das Gerät dazu konfiguriert, eine Überprüfung und/oder Bestätigung eines korrekten Anschlusses zumindest eines der in Verwendung befindlichen Konzentratgebinde von einem Anwender anzufordern.

Die Erfindung wird nachstehend anhand eines bevorzugten Ausführungsbeispiels unter Bezugnahme auf die beigefügte Zeichnung näher beschrieben. Es zeigen:
Fig. 1 schematisch vereinfacht und ausschnittsweise eine grundlegende Anordnung einer Konzentratversorgung in einem Gerät zur extrakorporalen Blutbehandlung;
Fig. 2 schematisch vereinfacht und ausschnittsweise eine Anordnung einer Konzentratversorgung in einem Gerät zur extrakorporalen Blutbehandlung gemäß einem ersten Ausführungsbeispiel des für einen automatischen Konzentratwechsel konfigurierten Geräts zur extrakorporalen Blutbehandlung; und
Fig. 3 schematisch vereinfacht und ausschnittsweise eine Anordnung einer Konzentratversorgung in einem Gerät zur extrakorporalen Blutbehandlung gemäß einem zweiten Ausführungsbeispiel des für einen automatischen Konzentratwechsel konfigurierten Geräts zur extrakorporalen Blutbehandlung.

In der nachfolgenden Figurenbeschreibung können in einzelnen Figuren gleiche oder gleichwirkende Elemente und/oder Komponenten gleich und/oder mit denselben Bezugszeichen bezeichnet und zweckmäßig nicht redundant beschrieben sein. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

Ein Gerät zur extrakorporalen Blutbehandlung, wie beispielsweise eine Dialysemaschine zur Reinigung von Blut eines Patienten, wenn z.B. dessen Nierenfunktion eingeschränkt oder eingestellt ist, weist einen Dialysator auf, der einerseits von dem zu reinigenden Blut des Patienten und andererseits von einer Dialysierflüssigkeit oder Dialyselösung, vorzugsweise gemäß dem Gegenstromprinzip, durchströmt wird, wobei gewisse gelöste Substanzen (z.B. Harnstoff) aus dem Blut an die Dialysierflüssigkeit übergehen.

Systematik, Aufbau, Komponenten und Funktionsweise des vorgenannten Geräts zur extrakorporalen Blutbehandlung, das insbesondere ein Dialysegerät oder eine Dialysemaschine sein kann, sind an sich grundlegend bekannt und werden daher diesbezüglich hierin einbezogen und nicht weiter beschrieben.

Ein solches Gerät verfügt vorzugsweise weiter über eine vorbestimmte Anzahl von Anschlüssen, über welche ein Säurekonzentrat und/oder ein Bicarbonatkonzentrat zur Herstellung benötigter Dialysierflüssigkeit zuleitbar ist. Mit den einzelnen Anschlüssen sind in der Regel Gebinde 10 vorbestimmter Art, wie beispielsweise PE-Kanister, Schlauchleitungen zu größeren Konzentratvorräten oder flexible Kunststoffbeutel oder Kartuschen verbindbar. Flüssiges Konzentrat kann beispielsweise mittels zumindest einer Ansauglanze aus einem Gebinde 10 (Kanister, Beutel) geeigneter Art und Größe gefördert und dem Gerät zugeführt werden. In beutel- oder kartuschenförmigen Gebinden vorgehaltenes Trockenkonzentrat kann beispielsweise unmittelbar vor Gebrauch direkt an dem Gerät gelöst werden. Die Konzentratzufuhr zu dem Gerät kann darüber hinaus in eine an sich bekannte zentrale Konzentratversorgung des Geräts integriert sein.

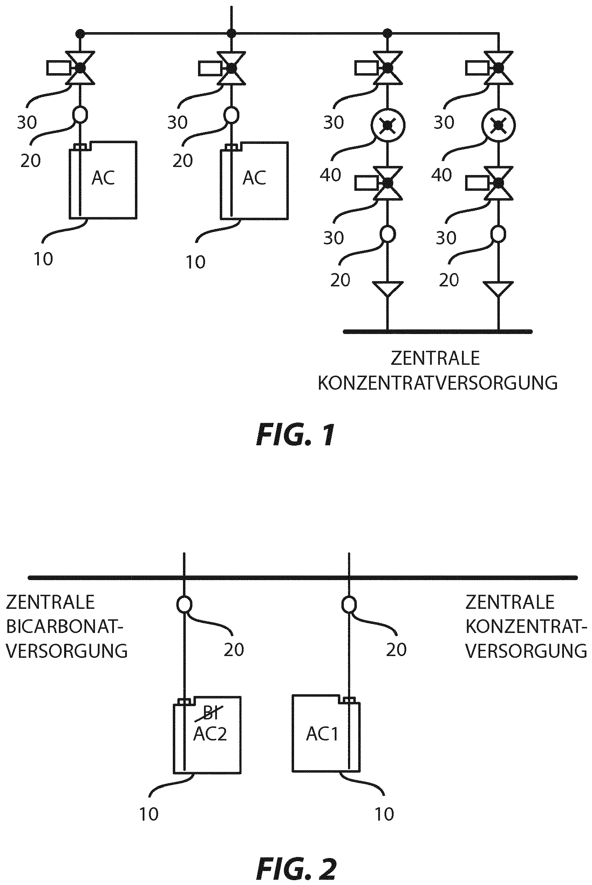

Fig. 1 zeigt schematisch vereinfacht und ausschnittsweise eine grundlegende Anordnung einer Konzentratversorgung in einem Gerät zur extrakorporalen Blutbehandlung, wie sie in nachfolgenden Ausführungsbeispielen angeordnet sein kann.

Gemäß Fig. 1 können an dem Gerät zur extrakorporalen Blutbehandlung zumindest zwei Säurekonzentratanschlüsse zur Ankopplung zumindest zweier Säurekonzentrat-Vorratsbehälter oder -gebinde 10 vorgesehen sein. In jeden Säurekonzentratvorrat in den Gebinden 10 kann eine Ansauglanze eintauchen, mittels welcher eine vorbestimmte Konzentratmenge aus dem Vorrat förderbar und dem Gerät zur extrakorporalen Blutbehandlung bzw. einer zentralen Konzentratversorgung desselben zuleitbar ist. In den entsprechenden Fluidleitungen können Konzentratfilter 20, Ventile 30, Pumpen 40 und dergleichen angeordnet sein, die zur Förderung benötigter Konzentratmengen geeignet ausgelegt und ansteuerbar sein können. Eine Trockenkonzentratversorgung kann mit artspezifischen Unterschieden, beispielsweise bezüglich eines Abschnitts zur Lösung des Trockenkonzentrats am Gerät, in vergleichbarer Weise aufgebaut sein.

Fig. 2 zeigt schematisch und ausschnittsweise eine Anordnung einer Konzentratversorgung in einem Gerät zur extrakorporalen Blutbehandlung gemäß einem ersten Ausführungsbeispiel des für einen Konzentratwechsel konfigurierten Geräts zur extrakorporalen Blutbehandlung.

Ein bestehendes Gerät zur extrakorporalen Blutbehandlung kann beispielsweise über jeweils einen Anschluss für ein Bicarbonatkonzentrat (BI), an welchem Bicarbonat oder Bicarbonatkonzentrat aus dem Vorrat oder Gebinde vermittels einer zentralen Bicarbonatversorgung des Geräts zuleitbar ist, und einen Anschluss für ein erstes Säurekonzentrat (AC1), an welchem das erste Säurekonzentrat aus dem Vorrat oder Gebinde 10 vermittels einer zentralen Konzentratversorgung des Geräts zuleitbar ist, aufweisen.

Erfindungsgemäß sind zumindest zwei Säurekonzentratanschlüsse vorgesehen, um ein erstes (AC1) und ein zweites (AC2) Säurekonzentrat an dem Gerät vorhalten zu können, zwischen welchen ein Wechsel während der laufenden Therapie oder Blutbehandlung einsteuerbar ist.

Eine automatisierte zentrale Konzentratversorgung kann beispielsweise mit zumindest drei Anschlüssen mit jeweils Ansauglanzen- bzw. Ansaugstabzugang konfiguriert sein. In diesem Fall kann eine Änderung der Hardwarekonfiguration entfallen, und sind an einem ersten Zugang ein erstes Konzentrat und an einem zweiten Zugang ein zweites Konzentrat bereitstellbar, wobei an einem dritten Zugang weiter Bicarbonatkonzentrat bereitstellbar ist.

Gemäß dem in Fig. 2 dargestellten ersten Ausführungsbeispiel ist bei einem Gerät mit ursprünglich zwei Ansaugstabzugängen der Anschluss für die Bicarbonatversorgung des Geräts als Anschluss für ein zweites Säurekonzentrat genutzt und konfiguriert ( → AC2), und, da insoweit der Ansaugstabzugang für ursprünglich Bicarbonat entfällt, das Gerät konstruktiv zur Versorgung mit Bicarbonat aus anderweitig bereitgestellten Gebinden wie beispielsweise Beuteln oder Kartuschen (nicht dargestellt) konfiguriert bzw. erweitert.

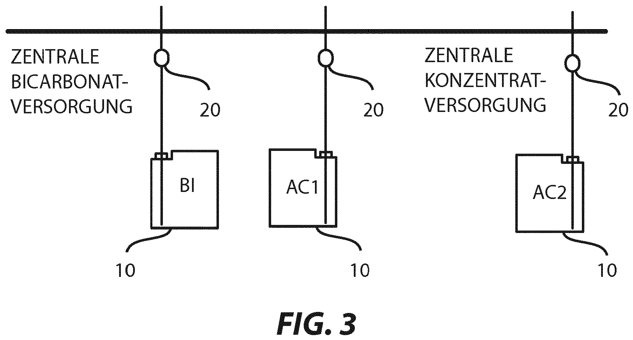

Fig. 3 zeigt schematisch vereinfacht und ausschnittsweise eine Anordnung einer Konzentratversorgung in einem Gerät zur extrakorporalen Blutbehandlung gemäß einem zweiten Ausführungsbeispiel des für einen automatischen Konzentratwechsel konfigurierten Geräts zur extrakorporalen Blutbehandlung.

Gemäß dem in Fig. 3 dargestellten zweiten Ausführungsbeispiel wurde ein Gerät mit ursprünglich zwei Ansaugstabzugängen, dargestellt linksseitig in Fig. 3, um zumindest einen weiteren Anschluss für zumindest ein zweites Säurekonzentrat (AC2), dargestellt rechtsseitig in Fig. 3, erweitert. Die ursprüngliche Bicarbonatversorgung (BI) und Säurekonzentrationsversorgung mit einem ersten Säurekonzentrat (AC1) des Geräts bleiben erhalten. Damit werden insgesamt drei Anschlüsse und/oder Ansaugstäbe bereitgestellt, wobei in dem vorliegenden zweiten Ausführungsbeispiel nur die zumindest zwei Säureanschlüsse AC1, AC2 entsprechend automatisch umschalten oder wechseln.

Darüber hinaus kann eine erweiterte Konfiguration der Steuerung und/oder Ansteuerung mit Unterstützung für den Anwender derart vorgesehen sein, dass an dem Gerät zumindest ein erstes und ein zweites Konzentrat bzw. eine erste und eine zweite Konzentratmenge vorbestimmbar und eingebbar sind, und in Übereinstimmung mit einer Vorgabe oder Voreinstellung eines Zeitpunkts ein durch das Gerät dann eigengesteuerter bzw. automatischer Wechsel von dem ersten Konzentrat auf das zweite Konzentrat durchgeführt wird.

Im entsprechenden Therapiekontext kann dazu ferner eine Patientenkarten- und/oder Netzwerkparameter-Eingabe derart vorgesehen sein, das ein Therapiesystem jederzeit zumindest einen Therapiefortschritt-Wechselzeitpunkt und/oder eine Konzentratauswahl anzeigt.

Eine grundlegende Funktions- und Arbeitsweise der vorgenannten Einrichtung in Verbindung mit dem Gerät zur extrakorporalen Blutbehandlung kann wie folgt konfiguriert sein.

Nach einem initialisierenden Eingabevorgang durch beispielsweise Technikpersonal sind in dem Gerät zur extrakorporalen Blutbehandlung alle verwendeten bzw. verwendbaren Konzentrate abgelegt oder abgespeichert und damit dem Gerät bekannt.

Während Vorbereitungsmaßnahmen und/oder eines Selbsttests wird über Mischungsverhältnisse eine Überprüfung auf Richtigkeit, beispielsweise auf korrekte Art und/oder ordnungsgemäßen Anschluss, und Patientenbezogenheit des Konzentrats und/oder Konzentratanschlusses durchgeführt. Der Anwender bestätigt vor Therapiebeginn die Korrektheit der Auswahl der Konzentrate und die Belegung der Anschlüsse durch die Konzentrate.

Ein während einer vorangehenden Therapie protokollierter Konzentratwechselzeitpunkt kann ausgelesen und als Vorgabewert für eine nächste oder nachfolgende Therapie herangezogen werden, d.h. ein solcher Wechselzeitpunkt steht geräteseitig als Vorgabe für die nächste Therapie zur Verfügung.

Ist im weiteren Verlauf die Therapie begonnen, kann das System bzw. das Gerät dazu eingerichtet sein, jeweils patientenbezogene Konzentrate und deren (Mischungs- oder Verdünnungs) Verhältnisse automatisch zu lernen und in einer dazu vorgesehenen Speichereinrichtung abzuspeichern.

Ferner kann vorgesehen sein, dass das System bzw. das Gerät bei Erfassung einer Mischungsverhältnisänderung den Anwender dazu befragt und dass der Anwender nach zusätzlicher beispielsweise visueller Überprüfung den richtigen oder korrekten Anschluss des Konzentratgebindes zu bestätigen hat, auf der Grundlage des durch das Gerät berechneten Mischungsverhältnisses (Ratio). Ein richtiger oder korrekter Anschluss des Konzentratgebindes kann sich hierbei insbesondere auf einen im Sinne von fehlerfreien und ordnungsgemäßen Anschluss an das Gerät bzw. eine solche Verbindung zu oder mit dem Gerät beziehen.

Es versteht sich, dass die Erfindung nicht auf die beschriebenen Ausführungsbeispiele und dessen Modifikationen beschränkt ist, sondern dass der Schutzumfang durch die nachstehenden Ansprüche definiert wird.

## Patentansprüche

1. Gerät zur extrakorporalen Blutbehandlung mit
zumindest einem ersten Säurekonzentratanschluss, der dazu angeordnet ist, zumindest ein erstes Säurekonzentrat (AC1) als Grundlage für eine Erzeugung einer Dialysierflüssigkeit in das Gerät zur extrakorporalen Blutbehandlung zu leiten; und
zumindest einem zweiten Säurekonzentratanschluss, der dazu angeordnet ist, zumindest ein zweites Säurekonzentrat (AC2) als Grundlage für eine Erzeugung einer Dialysierflüssigkeit in das Gerät zur extrakorporalen Blutbehandlung zu leiten; wobei
das Gerät zur extrakorporalen Blutbehandlung dazu eingerichtet ist, während einer laufenden Blutbehandlung zu einem vorbestimmten Zeitpunkt oder nach einer vorbestimmten Zeitspanne von einer Zufuhr des zumindest ersten Säurekonzentrats (AC1) auf eine Zufuhr des zumindest zweiten Säurekonzentrats (AC2) umzuschalten,
wobei ein Bicarbonatanschluss des Geräts derart als der erste oder der zweite Konzentratanschluss konfiguriert ist, dass dem Gerät über den Bicarbonatanschluss das erste oder das zweite Säurekonzentrat (AC1, AC2) zuführbar ist, und Bicarbonat (BI) separat von dem Bicarbonatanschluss zuführbar ist,
**dadurch gekennzeichnet, dass**
das Gerät dazu konfiguriert ist, einen Konzentratwechselzeitpunkt während einer laufenden Behandlung zu protokollieren und als Vorgabezeitpunkt für mindestens eine nachfolgende Behandlung zu speichern und bereitzustellen.

2. Gerät zur extrakorporalen Blutbehandlung nach Anspruch 1, bei dem das Gerät dazu angeordnet ist, die Umschaltung von der Zufuhr des zumindest ersten Säurekonzentrats (AC1) auf die Zufuhr des zumindest zweiten Säurekonzentrats (AC2) automatisch durchzuführen.

3. Gerät zur extrakorporalen Blutbehandlung nach einem der Ansprüche 1 oder 2, bei dem nur als Säurekonzentratanschlüsse konfigurierte Konzentratanschlüsse dazu angeordnet sind, automatisch umzuschalten.

4. Gerät zur extrakorporalen Blutbehandlung nach einem der Ansprüche 1 bis 3, bei dem das erste Säurekonzentrat (AC1) und/oder das zweite Säurekonzentrat (AC2) in Form eines Flüssigkonzentratvorrats oder Trockenkonzentratvorrats in jeweils einem ersten und einem zweiten Konzentratgebinde (10) vorhaltbar sind, wobei das erste Konzentratgebinde (10) konzentratdurchlässig mit dem ersten Säurekonzentratanschluss und das zweite Konzentratgebinde (10) konzentratdurchlässig mit dem zweiten Säurekonzentratanschluss koppelbar sind.

5. Gerät zur extrakorporalen Blutbehandlung nach einem der vorangehenden Ansprüche 1 bis 4, bei dem zusätzlich zu bestehenden Konzentratanschlüssen mindestens ein weiterer Konzentratanschluss als der erste oder der zweite Säurekonzentratanschluss angeordnet ist.

6. Gerät zur extrakorporalen Blutbehandlung nach einem der Ansprüche 1 bis 5, bei dem eine Vielzahl von Arten verwendbarer erster und zweiter Konzentrate vorab in einer Speichereinrichtung des Geräts gespeichert sind, und an dem Gerät zumindest das erste und zumindest das zweite Säurekonzentrat (AC1, AC2) aus der dem Gerät bekannten Vielzahl von Arten verwendbarer Konzentrate auswählbar sind.

7. Gerät zur extrakorporalen Blutbehandlung nach einem der Ansprüche 1 bis 6, bei dem das Gerät dazu konfiguriert ist, zumindest eine patientenbezogen korrekte Auswahl von Konzentraten, Mischungsverhältnisse derselben und eine Anschlussbelegung durch ausgewählte Konzentrate über eine Mehrzahl von Behandlungen hinweg zu lernen und zu speichern.

8. Gerät zur extrakorporalen Blutbehandlung nach einem der Ansprüche 1 bis 7, bei dem dann, wenn geräteseitig eine Änderung eines konzentratbezogenen Mischungsverhältnisses erfasst wird, das Gerät dazu konfiguriert ist, eine Überprüfung und/oder Bestätigung eines korrekten Anschlusses zumindest eines der in Verwendung befindlichen Konzentratgebinde anzufordern.

## Claims

1. Device for extracorporeal blood treatment, comprising:
at least a first acid concentrate connection which is configured to feed at least a first acid concentrate (AC1) into the device for extracorporeal blood treatment as a basis for generating a dialysate; and
at least a second acid concentrate connection which is configured to feed at least a second acid concentrate (AC2) into the device for extracorporeal blood treatment as a basis for generating a dialysate; wherein
the device for extracorporeal blood treatment is configured to switch over at a predetermined time during an ongoing blood treatment or after a predetermined period of time from feeding the at least first acid concentrate (AC1) to feeding the at least second acid concentrate (AC2),
wherein a bicarbonate connection of the device is configured as the first or the second acid concentrate connection in such a way that the first or the second acid concentrate (AC1, AC2) can be supplied to the device via the bicarbonate connection, and bicarbonate (BI) can be supplied separately from the bicarbonate connection,
**characterized in that**
the device is configured to protocol a concentrate changeover time in the course of an ongoing treatment and to save and make this available as a predefined time for at least one subsequent treatment.

2. Device for extracorporeal blood treatment according to claim 1, wherein the device is configured to carry out the changeover from supplying the at least first acid concentrate (AC1) to supplying the at least second acid concentrate (AC2) automatically.

3. Device for extracorporeal blood treatment according to one of claims 1 or 2, wherein only concentrate connections configured as acid concentrate connections are configured to automatically switch over.

4. Device for extracorporeal blood treatment according to one of claims 1 to 3, wherein the first acid concentrate (AC1) and/or the second acid concentrate (AC2) can be provided in the form of a liquid concentrate supply or dry concentrate supply in a first and a second acid concentrate (AC2) container respectively, wherein the first acid concentrate (AC1) container is capable of being connected to the first acid concentrate (AC1) connection in a way which is permeable to concentrate and the second acid concentrate (AC2) container is capable of being connected to the second acid concentrate (AC2) connection in a way which is permeable to concentrate.

5. Device for extracorporeal blood treatment according to one of the preceding claims 1 to 4, wherein in addition to existing concentrate connections, at least one further concentrate connection is provided as the first or the second acid concentrate connection.

6. Device for extracorporeal blood treatment according to one of the preceding claims 1 to 5, wherein a large number of types of usable first and second acid concentrates are stored in advance in a storage facility of the device, and it is possible to select at the device at least the first acid concentrate (AC1) and at least the second acid concentrate (AC2) from the large number of usable concentrates known to the device.

7. Device for extracorporeal blood treatment according to one of the preceding claims 1 to 6, wherein the device is configured to learn and save at least one correct patient-related selection of concentrates, mixture ratios of the same and a connection assignment to selected concentrates over the course of several treatments.

8. Device for extracorporeal blood treatment according to one of the preceding claims 1 to 7, wherein when a change in the concentrate-related mixing ratio is registered by the device, the device is configured in such a way as to request a verification and/or confirmation of a correct connection of at least one of the concentrate containers being used.

## Revendications

1. Appareil de traitement extracorporel du sang avec
au moins un premier raccord de concentré d'acide qui est agencé afin d'acheminer au moins un premier concentré d'acide (AC1) comme base pour une génération d'un liquide de dialyse dans l'appareil pour le traitement extracorporel du sang ; et
au moins un second raccord de concentré d'acide qui est agencé afin d'acheminer au moins un second concentré d'acide (AC2) comme base pour une génération d'un liquide de dialyse dans l'appareil de traitement extracorporel du sang ; dans lequel
l'appareil de traitement extracorporel du sang est conçu afin de commuter pendant un traitement du sang en cours à un moment prédéterminé ou après un intervalle prédéterminé d'une alimentation de l'au moins premier concentré d'acide (AC1) à une alimentation de l'au moins second concentré d'acide (AC2),
dans lequel un raccord de bicarbonate de l'appareil est configuré comme le premier ou le second raccord de concentré de telle manière que le premier ou le second concentré d'acide (AC1, AC2) puisse être alimenté dans l'appareil par le biais du raccord de bicarbonate, et du bicarbonate (BI) puisse être alimenté séparément du raccord de bicarbonate,
**caractérisé en ce que**
l'appareil est configuré afin d'enregistrer un moment de changement de concentré pendant un traitement en cours et d'enregistrer et de le fournir comme moment de prescription pour au moins un traitement suivant.

2. Appareil de traitement extracorporel du sang selon la revendication 1, pour lequel l'appareil est agencé afin de réaliser automatiquement la commutation de l'alimentation de l'au moins premier concentré d'acide (AC1) à l'alimentation de l'au moins le second concentré d'acide (AC2).

3. Appareil de traitement extracorporel du sang selon l'une quelconque des revendications 1 ou 2, pour lequel seuls des raccords de concentré configurés comme raccords de concentré d'acide sont agencés afin de commuter automatiquement.

4. Appareil de traitement extracorporel du sang selon l'une quelconque des revendications 1 à 3, pour lequel le premier concentré d'acide (AC1) et/ou le second concentré d'acide (AC2) peuvent être conservés sous la forme d'une réserve de concentré liquide ou réserve de concentré sec dans respectivement un premier et un second récipient de concentré (10), dans lequel le premier récipient de concentré (10) est couplable de manière perméable au concentré au premier raccord de concentré d'acide et le second récipient de concentré (10) est couplable de manière perméable au concentré au second raccord de raccord d'acide.

5. Appareil de traitement extracorporel du sang selon l'une quelconque des revendications 1 à 4 précédentes, pour lequel au moins un autre raccord de concentré est agencé outre des raccords de concentré existants comme le premier ou le second raccord de concentré d'acide.

6. Appareil de traitement extracorporel du sang selon l'une quelconque des revendications 1 à 5, pour lequel une pluralité de types de premiers et seconds concentrés utilisables sont enregistrés au préalable dans un dispositif de mémorisation de l'appareil, et au niveau de l'appareil au moins le premier et au moins le second concentré d'acide (AC1, AC2) peuvent être sélectionnés parmi la pluralité connue de l'appareil de types de concentrés utilisables.

7. Appareil de traitement extracorporel du sang selon l'une quelconque des revendications 1 à 6, pour lequel l'appareil est configuré afin d'apprendre et d'enregistrer au moins une sélection correcte relative au patient de concentrés, rapports de mélange de ceux-ci et une occupation de raccord par des concentrés sélectionnés sur une pluralité de traitements.

8. Appareil de traitement extracorporel du sang selon l'une quelconque des revendications 1 à 7, pour lequel lorsqu'une modification d'un rapport de mélange relatif au concentré est détectée côté appareil, l'appareil est configuré afin d'exiger une vérification et/ou validation d'un raccord correct au moins d'un des récipients de concentré se trouvant en utilisation.
